# EUROPEAN PATENT SPECIFICATION

(11) **EP 4 483 187 B1**
(45) Date of publication and mention of the grant of the patent: **03.12.2025**
(21) Application number: 23707424.0
(22) Date of filing: 08.02.2023
(51) Int. Cl.: G01N 33/68

(54) **A METHOD FOR DIAGNOSIS OF TRAUMATIC BRAIN INJURY**
VERFAHREN ZUR DIAGNOSE TRAUMATISCHER HIRNVERLETZUNGEN
MÉTHODE DE DIAGNOSTIC D'UN TRAUMATISME CRÂNIO-CÉRÉBRAL

(30) Priority: 22.02.2022 FI 20225157
(43) Date of publication of application: 01.01.2025
(73) Proprietor: Medicortex Finland Oyj, 20520 Turku (FI)
(72) Inventor: VÄLIMAA, Lasse, 20520 Turku (FI); HAREL, Adrian, 20520 Turku (FI); HAAVISTO, Oskar, 20520 Turku (FI); KVIST, Mårten, 20520 Turku (FI)
(74) Representative: Laine IP Oy
(86) International application number: PCT/FI2023/050076
(87) International publication number: WO 2023/161557

(56) References cited:
- EP-A2- 3 757 226
- WO-A2-2021/262905
- US-A1- 2010 028 913
- US-A1- 2019 242 906
- US-A1- 2021 311 043
- KVIST MÅRTEN ET AL: "Glycans as Potential Diagnostic Markers of Traumatic Brain Injury", BRAIN SCIENCES, vol. 11, no. 11, 9 November 2021 (2021-11-09), pages 1480, XP093018765, DOI: 10.3390/brainsci11111480
- RAYBIOTECH INC ET AL: "RayBiotech Lectin Array 70 User Manual (Version 3) Cat # GA-Lectin-70", 4 October 2021 (2021-10-04), pages 770 - 206, XP093042403, Retrieved from the Internet <URL:https://www.raybiotech.com/lectin-array-70-ga-lectin-70#tab_documents> [retrieved on 20230426]

## Description

### FIELD

The present disclosure relates to an *in vitro* method for diagnosing traumatic brain injury (TBI), in particular to methods wherein the diagnosis is based on determining level of certain glycan-based biomarkers such as glycoproteins and cleavage products thereof in body fluid of a subject suspected to suffer from TBI. The disclosure also relates to use of N-glycans as biomarkers of TBI.

### BACKGROUND

Traumatic brain injury (TBI) is a multifaceted condition caused by an external force to the head that occurs in, e.g., accidents in traffic, at home, at work, in sports, and on the battlefield. It is broadly defined as an alteration in brain function or other evidence of brain pathology.

Early recognition of brain injury is vital in the process of controlling TBI and starting proper care in order to secure positive prognosis for the patient. Currently, the first diagnostic measure is neurological examination based on patient's verbal response, eye movement, and motion response (Glasgow Coma Scale, GCS). However, this is not very accurate, as the symptoms of TBI are subjective, or because similar symptoms of different origin such as intoxication may overwhelm the signs of TBI. More advanced diagnostic measures in wide use are head scanning by computed tomography (CT) or by magnetic resonance imaging (MRI). However, in many cases mild TBI (concussion) does not result in detectable alterations in the CT or MRI. Moreover, scanners are expensive big devices, and they are typically available in well-equipped hospitals only. Thus, concussion diagnosis relies significantly on GCS and patient's history.

Molecular methods such as biomarker-based diagnostic tools offer a complementary or a surrogate method to detect TBI. They can offer a robust standardized tool for objective classification of TBI. They even have potential to predict the outcome (prognosis) or monitor the state of recovery. Additionally, biomarker-based approaches bear potential for reducing the time and costs of diagnosis.

Several TBI specific blood and cerebrospinal fluid (CSF) biomarker proteins have received considerable research. Examples of these include Ubiquitin C-terminal hydrolase-L1 (UCH-L1), Neuron specific enolase (NSE), Glial fibrillary acidic protein (GFAP), S100 calcium-binding protein B (S100β), Spectrin breakdown product (SBDP), and hyperphosphorylated neurofilaments (p-NF). The practical use that a few biomarkers have seen until today is a reduction in CT requirement among patients who are entering the emergency room with suspected mild brain injury (negative predictive value). To date, they have not been implemented for distinguishing between cases with TBI and without TBI.

Mårten Kvist, et al, Brain Sciences, 2021, 11, 1480 teach glycan biomarkers for the diagnosis of traumatic brain injury.

Accordingly, there is need for more specific biomarkers for traumatic brain injury.

### SUMMARY

The present invention is based on the observation that increase of concentration of certain glycan-based biomarkers in urine and/or saliva can be regarded as an indication of traumatic brain injury. The scope of the invention is defined by the appended claims. Embodiments and descriptions no longer falling under the scope of the appended claims are considered merely as examples suitable for understanding the invention.

Accordingly, it is an object of the present invention to provide a new *in vitro* method of diagnosing traumatic brain injury (TBI) in a subject, the method comprising the steps of:
a) providing urine and/or saliva sample of said subject,
b) determining level of binding of at least one glycan-based biomarker in said urine and/or saliva sample to at least one lectin,
c) comparing the determined level of binding to two control levels of said glycan-based biomarker, wherein said control levels are
   - level of binding of said at least one glycan-based biomarker to said at least one lectin in saliva and/or urine of an uninjured subject and
   - level of binding of said at least one glycan-based biomarker to said at least one lectin in saliva and/or urine of a subject suffering from orthopaedic injury, and
d) providing a diagnosis based on said comparing, wherein ≥20% increased level of binding of said at least glycan-based biomarker compared the two control levels is indicative of TBI in said subject wherein
   - for urine sample, the at least one lectin is selected from a group consisting of: UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I). GS-I (GSL-I, BSL-I), PSA (PEA), LENTIL, LEA (LEL, TL), F17AG, LcH (LCA) and DISCOIDIN I, and
   - for saliva sample the at least one lectin is selected from a group consisting of: PTL-1 (PTL, WBA-I), CNL, GAL7-S and CSA.

It is also an object of the present invention to provide a new *in vitro* method of diagnosing traumatic brain injury (TBI) in a subject, the method comprising the steps of:
a) providing a urine and/or a saliva sample obtained from the subject,
b) determining level of at least one glycan-based biomarker in said urine and/or saliva sample,
c) comparing the determined level to a control level of said glycan-based biomarker, wherein said control level is the level of said biomarker in saliva and/or urine of an uninjured subject and
d) providing a diagnosis based on said comparing, wherein ≥20% increased level of said at least glycan-based biomarker compared the control level is indicative of TBI in said subject wherein
   - for the urine sample, the at least one glycan-based biomarker comprises a N-glycan selected from a group consisting of H4N2F1, H3N2, H9N2, H4N3F1S1, and H4N3F3S1, and/or
   - for the saliva sample, the at least one glycan-based biomarker comprises a N-glycan selected from a group consisting of H5N4F2P1, H5N4F3P1, H5N5F1S1, and H3N2F1, wherein
      H4N2F1 is 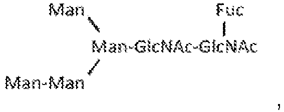
      H3N2 is 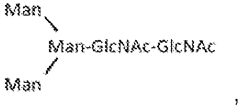
      H9N2 is 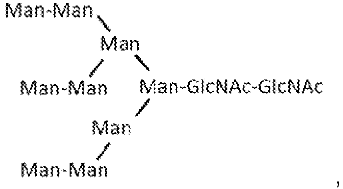
      H4N3F1S1 is 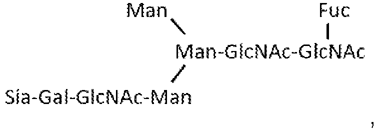
      H4N3F3S1 is 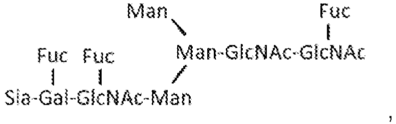
      H5N4F2P1 is selected from 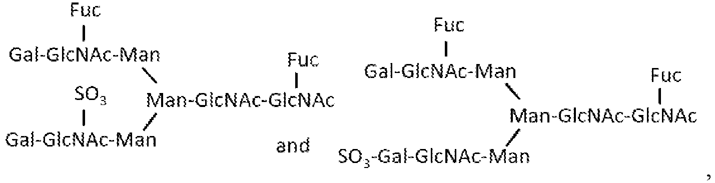
      H5N5F1S1 is 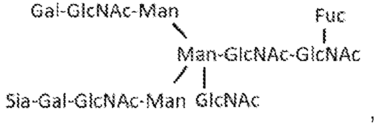
      H3N2F1 is 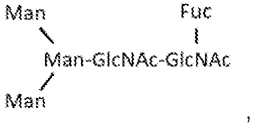 and
      H5N4F3P1 is selected from a group consisting of

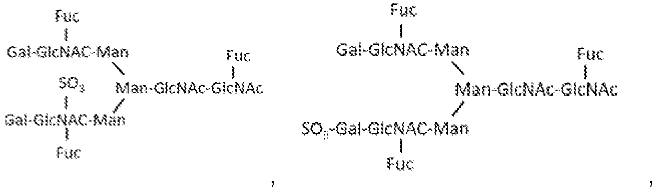

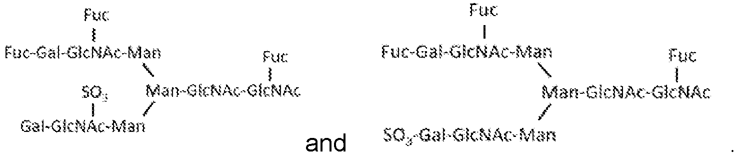

It is also an object of the present invention to provide a new use of at least one glycan-based biomarker comprising a N-glycan selected from a group consisting of H4N2F1, H3N2, H9N2, H4N3F1S1, and H4N3F3S1, wherein
H4N2F1 is 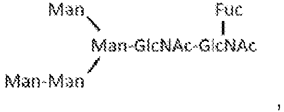
H3N2 is 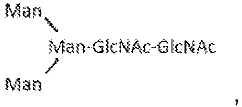
H9N2 is 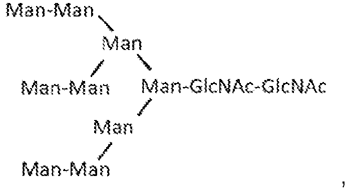
H4N3F1S1 is 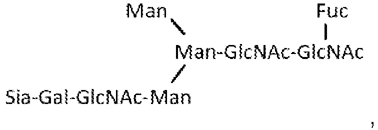 and
H4N3F3S1 is 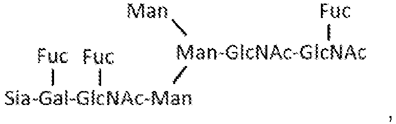 as an indicator of traumatic brain injury in urine.

It is also an object of the present invention to provide a new use of at least one glycan-based biomarker comprising a N-glycan selected from a group consisting of H5N4F2P1, H5N4F3P1, H5N5F1S1, and H3N2F1, wherein
H5N4F2P1 is selected from 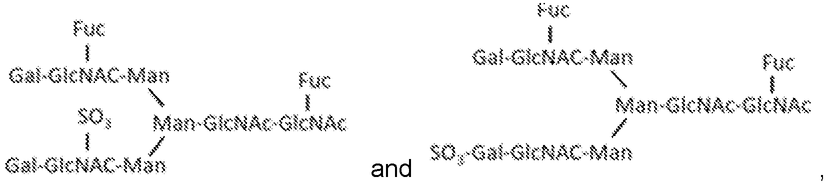 and 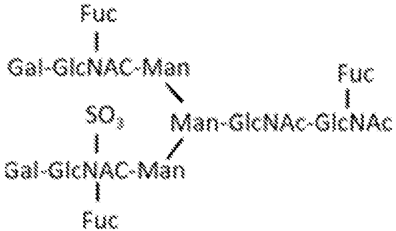
H5N4F3P1 is selected from , 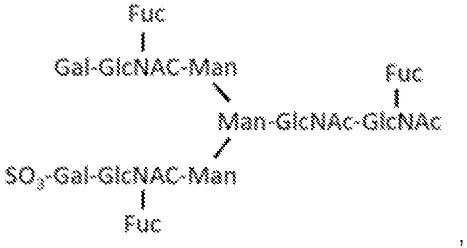 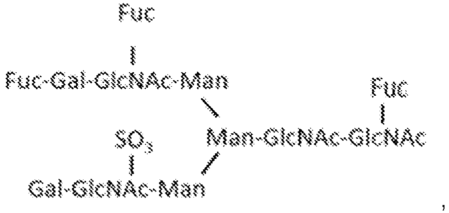 and 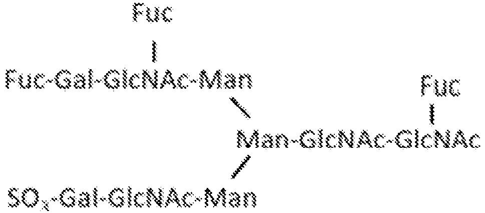
H5N5F1S1 is 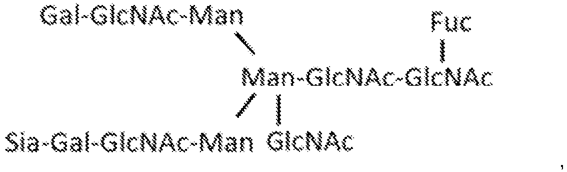 and

H3N2F1 is selected from the group consisting of
Manα1-3(Manα1-6)Manβ1-4GlcNAcβ1-4(Fucα1-6)GlcNAc,
Manα1-2Manα1-3Manβ1-4GlcNAcβ1-4(Fucα1-6)GlcNAc,
Manα1-3Manα1-6Manβ1-4GlcNAcβ1-4(Fucα1-6)GlcNAc, and
Manα1-6Manα1-6Manβ1-4GlcNAcβ1-4(Fucα1-6)GlcNAc, preferably 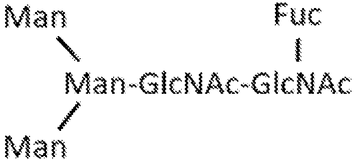
as an indicator of traumatic brain injury in saliva.

Further objects of the present invention are described in the accompanying dependent claims.

Exemplifying and non-limiting embodiments of the disclosure both as to constructions and to methods of operation, together with additional objects and advantages thereof, are best understood from the following description of specific exemplifying embodiments when read in connection with the accompanying drawings.

The verbs "to comprise" and "to include" are used in this document as open limitations that neither exclude nor require the existence of un-recited features. The features recited in the accompanied depending claims are mutually freely combinable unless otherwise explicitly stated. Furthermore, it is to be understood that the use of "a" or "an", i.e., a singular form, throughout this document does not exclude a plurality.

### BRIEF DESCRIPTION OF FIGURES

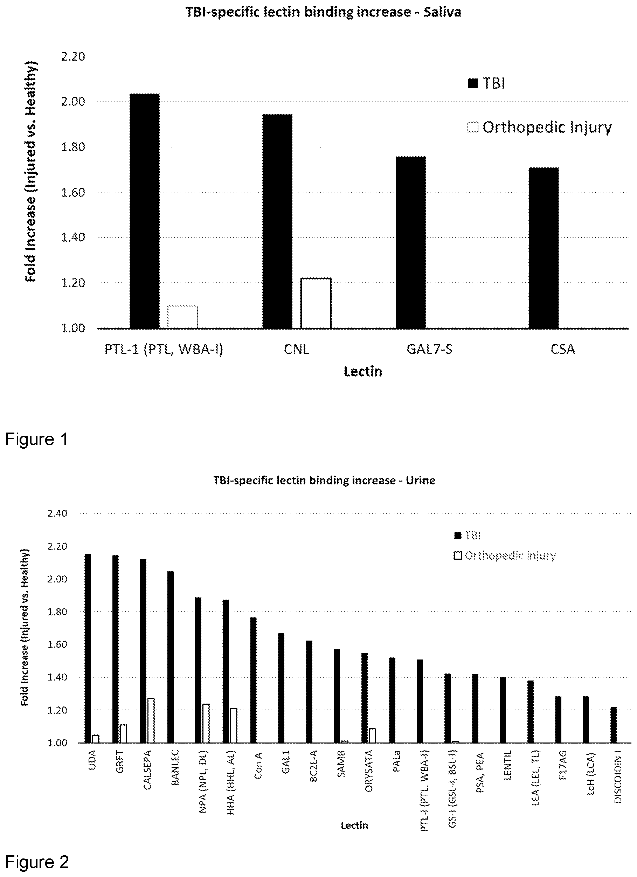

Figure 1 shows lectins with increase in binding of glycans in saliva samples. The y-axis shows the increase (fold change) in the average of injury samples compared to the average of the uninjured healthy control samples which is represented by the y-axis value of 1.00. Black bar: TBI; white bar: orthopaedic damage (shown when the ratio is ≥1.00).
Figure 2 shows lectins with increase in binding of glycans in urine samples. The y-axis shows the increase (fold change) in the average of injury samples compared to the average of the uninjured healthy control samples which is represented by the y-axis value of 1.00. Black bar: TBI; white bar: orthopaedic damage (shown when the ratio is ≥1.00).

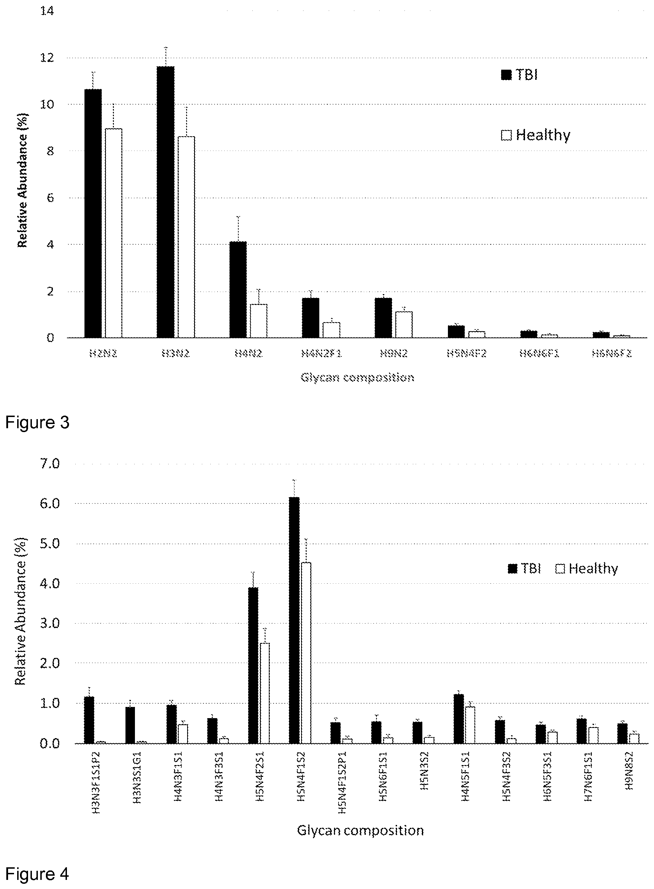

Figure 3 shows relative abundance of neutral N-glycans in urine as analysed by MALDI-TOF MS. The samples were pre-treated with ethanol precipitation and the ethanol precipitate was subjected to N-glycan detachment from sample glycoproteins with N-glycosidase F enzyme, followed by purification by solid phase extraction and mass spectrometry analysis. The bars show glycan structures of increased average relative abundance in TBI samples compared to uninjured healthy control samples.
Figure 4 shows relative abundance of acidic N-glycans in urine as analysed by MALDI-TOF MS. The samples were pre-treated with ethanol precipitation and the ethanol precipitate was subjected to N-glycan detachment from sample glycoproteins with N-glycosidase F enzyme, followed by purification by solid phase extraction and mass spectrometry analysis The bars show glycan structures of increased average relative abundance in TBI samples compared to uninjured healthy control samples.

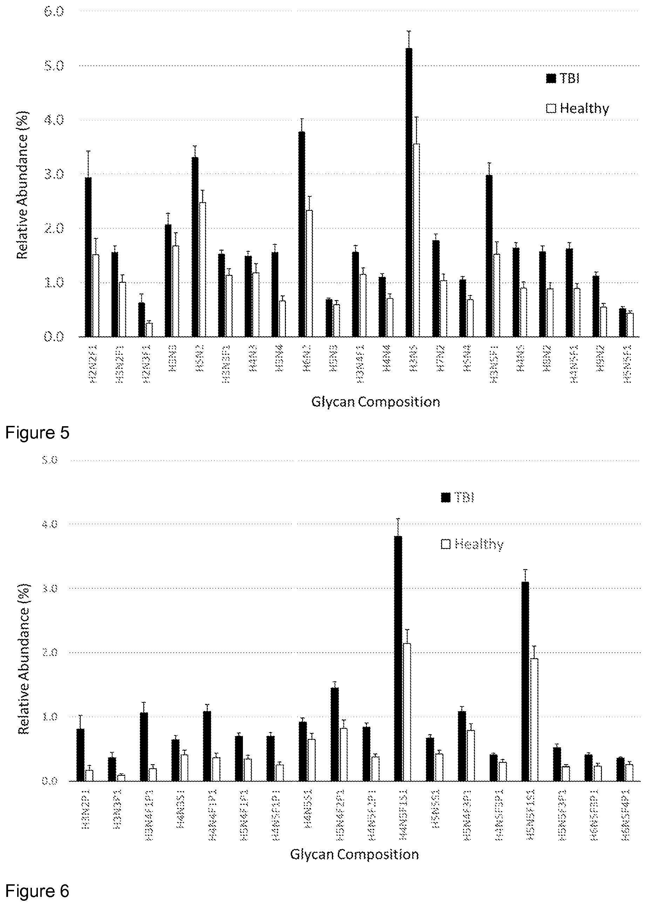

Figure 5 shows relative abundance of neutral N-glycans in saliva as analysed by MALDI-TOF MS. The samples were pre-treated with ethanol precipitation and the ethanol precipitate was subjected to N-glycan detachment from sample glycoproteins with N-glycosidase F enzyme, followed by purification by solid phase extraction and mass spectrometry analysis. The bars show glycan structures with increased average relative abundance in TBI samples (n = 76) compared to the average of uninjured healthy control samples (n = 28).
Figure 6 shows relative abundance of acidic N-glycans in saliva as analysed by MALDI-TOF MS. The samples were pre-treated with ethanol precipitation and the ethanol precipitate was subjected to N-glycan detachment from sample glycoproteins with N-glycosidase F enzyme, followed by purification by solid phase extraction and mass spectrometry analysis. The bars show glycan structures of increased average relative abundance in TBI samples (n = 76) compared to the average of uninjured healthy control samples (n = 28).

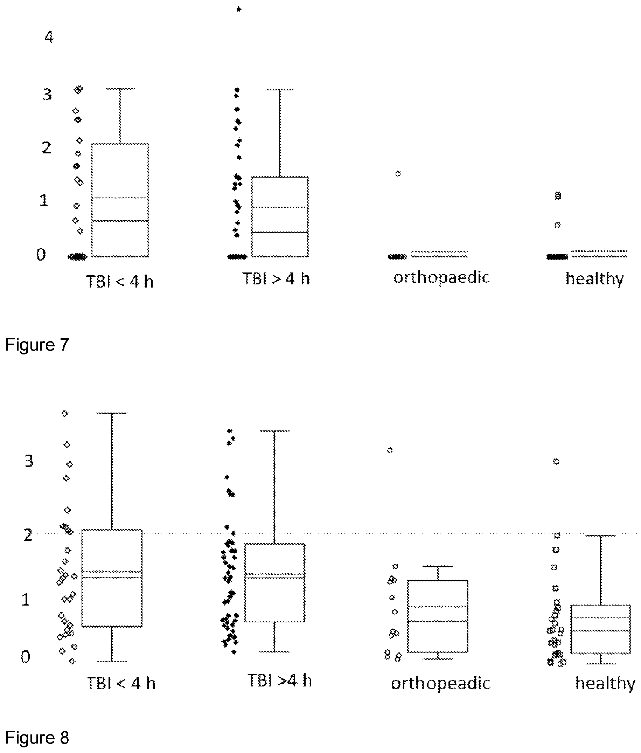

Figure 7 shows the level of H4N3F3S1 in urine samples obtained from subjects suffering from TBI, subjects suffering from orthopaedic injury, and healthy controls.
Figure 8 shows the level of H5N4F2P1 in saliva samples obtained from subjects suffering from TBI, subjects suffering from orthopaedic injury and healthy controls.

### DESCRIPTION

The present disclosure concerns an *in vitro* method of diagnosing traumatic brain injury (TBI) in a subject. The method comprises the following steps
i. determining level of at least one glycan-based biomarker in a body fluid sample of the subject,
ii. comparing the determined level of the glycan-based biomarker to its control level(s) in the body fluid, and
iii. providing the diagnosis based on the comparing, wherein increased level of the at least one glycan-based biomarker compared to control level(s) is indicative of TBI in the subject.

As defined herein, the term "biomarker" refers to a molecule that is detectable in a biological sample obtained from a subject and that is indicative of a brain damage in the subject. Markers of particular interest include glycan-based biomarkers showing differences in glycosylation between a sample from an individual with a brain damage, a sample from an individual with an orthopaedic damage, and a healthy control.

As defined herein, the term "glycan-based biomarker" refers to a polysaccharide, i.e. a polymer comprising two or more monosaccharide residues, as well as to a carbohydrate portion of a glycoconjugate, such as a glycoprotein, a glycolipid, a peptidoglycan, or a proteoglycan, or a fragment thereof. Glycan-based biomarkers may comprise either homo-polymeric or hetero-polymeric monosaccharide residues, and they may be either linear or branched. As used herein, the terms "glycan", "polysaccharide" and "carbohydrate" are interchangeable, unless otherwise indicated. The glycan-based biomarkers of suitable for the method bind lectins.

As defined herein "glycan" is an oligosaccharide or carbohydrate portion of a glycoconjugate, such as a glycoprotein, glycolipid, or a proteoglycan. The origin of the glycan can be cell content that is spilled and undergo metabolic degradation, enzymatic cleavage, or chemical breakdown. Such molecules can originate from the immune system like globulins or antibodies, blood proteins like albumin, or macromolecules originating from intracellular fluid.

As a biomarker, the glycan can occur as a mere glycan, or it can be a part of a glycoconjugate.

As defined herein, the term "diagnosis" refers to the determination of whether or not a subject has a TBI. The term is also meant to include instances where the presence of a brain damage is not finally determined but that further diagnostic testing is warranted. In such embodiments, the method is not by itself determinative of the presence or absence of a brain damage in the subject but can indicate that further diagnostic testing is needed or would be beneficial. The methods, therefore, can be combined with one or more other diagnostic methods for the final determination of the presence or absence of a brain damage in the subject. Examples of such other diagnostic methods include, but are not limited to, CT and MRI, and are well known to a person skilled in the art. As used herein, a "final determination" or "final diagnosis" refers to ascertaining the presence or absence of a brain damage in a subject. The final determination or final diagnosis can be the result of any of the methods of the invention which, in some embodiments, can include more than one diagnostic test.

As defined herein, the term "comparing" refers to assessing how the proportion, level, or cellular localization of one or more biomarkers in a sample from a subject relates to the proportion, level, or localization of the corresponding one or more biomarkers in a standard or control sample. For example, "comparing" may refer to assessing whether the proportion, level, or cellular localization of one or more biomarkers in a sample from a subject is the same as, more, or less than, or different from the proportion, level, or localization of the corresponding one or more biomarkers in standard or control sample. More specifically, the term may refer to assessing whether the proportion, level, or cellular localization of one or more biomarkers in a sample from a subject is the same as, more or less than, different from or otherwise corresponds (or not) to the proportion, level, or cellular localization of predefined biomarker levels that correspond to, for example, a subject having subclinical brain injury (SCI), not having SCI, is responding to treatment for SCI, is not responding to treatment for SCI, is/is not likely to respond to a particular SCI treatment, or having/not having another disease or condition. In a specific embodiment, the term "comparing" refers to assessing whether the level of one or more glycan-based biomarkers of the present invention in a sample from a subject is the same as, more, or less than, different from other otherwise correspond (or not) to levels of the same biomarkers in a control sample (e.g., predefined levels that correlate to individuals not suffering from TBI).

According to one embodiment the subject is a child. Children are the most common group suffering TBI. In addition, paediatric TBI is also the most challenging to diagnose. Youngest children, in particular, cannot reliably communicate their symptoms. The radiation exposure by CT scan is harmful for the vulnerable, developing young brain. TBI in children is fundamentally different; in children it is a chronic disease process rather than a one-time event. As defined herein a child is a subject of 0-17 years age.

According to another embodiment the subject is an old person. Elderly are at an increased risk of suffering TBI. They are prone to falls and accidents at home or in an elderly home. They are frail, thus small impacts and collisions can cause difficult injuries. A point-of-care test for TBI detection would be a great tool for assessing whether an injured person needs further medical intervention and transportation to the hospital, or not.

According to some embodiments the subject can be an animal, more specifically, a mammal.

The incidence rate for a TBI-related ED visit, hospitalization, or death is more than 1 in 200 among Americans at ages 65-74 years, and more than 1 in 50 at ages ≥75 years. Pre-existing conditions common in elderly, including past history of TBI, are further risk factors for suffering a TBI. Chronic diseases are also associated with worse outcome. Old people with pre-existing dementia may have an abnormal baseline GCS which further complicates the assessment of brain injury after a person has hurt his/her head.

The body fluid to be tested is selected from a group consisting of blood, cerebrospinal fluid, urine, saliva (spit, phlegm, sputum, nasal discharge), plasma, serum, lymph fluid, lymphatic fluid, interstitial fluid, tear, exudate, sweat and extracellular fluid, preferably urine, saliva, and plasma, more preferably urine and saliva.

The glycan-based biomarker suitable for the method is present and detectable in body fluid following a traumatic brain injury. The glycan-based biomarker can be in its intact, native conformation, in which case altered concentration of the glycan-based biomarker in body fluid, in comparison to healthy population, indicates injury. Alternatively, it can be an abnormal biodegradation product, in which case mere emergence of the abnormal product in the body fluid may indicate injury. As a consequence of cell damage, specific proteins are released that start to digest the surrounding proteins inexorably. This leads to the formation or elevation of certain glycan structures in bodily fluids that can indicate cell damage.

In certain embodiments, the glycan-based biomarker refer to a glycoprotein, i.e., a protein that has an oligosaccharide (glycan) group attached in post-translational modifications to an amino acid side chain. The glycan is attached via a glycosidic bond which forms between hemiacetal or hemiketal group of the saccharide and either hydroxyl, nitrogen, phosphorus, carbon, or sulphur group of the amino acid which are connoted O-, N-, P-, C- or S-glycosylation, respectively. Common glycosylated amino acids are asparagine, threonine, serine, tyrosine, tryptophan, and cysteine. The glycan-based biomarker can also be in the form a glycosylated fatty acid or glycolipid. For example, glycosphingolipids are present on cell surface membranes and are particularly abundant in the brain. Glycosphingolipids are a subtype of glycolipids containing an amino alcohol sphingosine. The glycan-based biomarker can also be a free-floating carbohydrate.

A screening of a panel of glycan-based biomarkers can give comprehensive information on the status of various brain tissues. It will help doctors and paramedics in targeting their preferred examination following diagnosis of a brain injury. For example, if the patient is unconscious, drunk, fainted, or unresponsive, the brain damage biomarker can serve as a means to easily obtain information on the condition of brain.

A glycan-based biomarker can also be used to monitor the effectiveness of an intervention or treatment, or healing of a specific brain tissue. These comprise monitoring the effect of a drug or medication, medical intervention like stem cells transplantation as well as perceiving spontaneously happening improvement or stabilization.

The origins of the glycan-based biomarker can originate from various brain cells (neurons, non-neuronal, glia, microglia, astrocytes, oligodendrocytes astrocytes), connective tissues, or blood vessels in the brain.

The glycan-based biomarkers can be found in any of the following body fluids:
- Blood (plasma or serum)
- Cerebrospinal fluid (CSF)
- Urine
- Saliva and other oral excretions (including also spit, mucus, sputum, phlegm, nasal discharge)
- Lymph fluid, lymphatic fluid, Interstitial fluid
- Tears
- Exudate
- Sweat
- Extracellular fluid

Especially, the glycan-based biomarker serves as a signal of brain injury, including
a) Acquired brain injury, ABI, brain injury that happens after the birth, including
   - Traumatic Brain Injury, TBI, due to external force, physical trauma, accidents, assaults, head injury, sports injury, impact to head, sudden movement of head, penetration of an object to head, etc.
   - Brain injury of non-traumatic origin (such as stroke, brain tumours, infection, poisoning, hypoxia, ischemia, encephalopathy, substance abuse, hypoxic and ischemic brain injury, neurosurgery, and medical operation, etc.).
      Embodiments of brain injury include, but are not limited to, inter-scalp bleeding, aneurysm, haemorrhage, cerebrovascular bleeding, intracranial haemorrhage, stroke, haemorrhagic stroke. In some embodiments, the glycan-based biomarker serves as a signal of the integrity of the Brain-Blood-Barrier (BBB).
b) Congenital (birth-related, prenatal, or inherited) disorder, such as fetal alcohol syndrome (FAS), partial fetal alcohol syndrome (pFAS), alcohol-related neurodevelopmental disorder (ARND), alcohol-related birth defects (ARBD), defects due to abuse of drugs during pregnancy, neonatal encephalopathy, neonatal hypoxic-ischemic encephalopathy, asphyxia neonatorum, birth asphyxia.

The glycan-based biomarker suitable for TBI analysis can have several sources. One possible origin is a glycoprotein or its cleavage product which is produced in the brain due to the release and uncontrolled function of various enzymes like proteases and glycosidases. For example, excess glutamate, an excitatory amino acid that elevates in the brain following TBI, leads to high levels of calcium ions which can influx into cells and can activate a number of enzymes, including phospholipases, endonucleases, and proteases such as calpain. These enzymes go on to damage cell structures such as components of the cytoskeleton, membrane, and organelles. Alternatively, the glycan-based biomarker can be related to abnormal glycosylation by glycosyltransferases that are released from the damaged brain cells (neuronal, non-neuronal, glia). The glycan-based biomarker can be released to the CSF and the blood flow through a breakage in the BBB and ultimately to saliva and urine.

Alternatively, the glycan-based biomarker can be related to an increase in the expression of proteins related to the physiological changes induced by TBI. It is known to induce cytokine and chemokine expression, and an increase in pro-inflammatory factors.

Upon brain injury the cellular contents are released into the surrounding liquid which is cerebrospinal fluid. From there, the molecule traverse through compromised blood-brain barrier (BBB) to the blood circulation and onward to urine and saliva. Thus, a glycan that is detected in urine or saliva is obviously present in the CSF and blood as well.

The structure can be with or without addition of one or more of the following: sialic acid, fucose, phosphate, sulphate, galactose. Their natural presence or absence is dependent on the conditions (whether acidic or basic environment). Additionally, the structures can rotate freely (for example, the two or more antennas can rotate with respect to the central core mannose).

According to a particular embodiment the glycan-based biomarkers are detected and/or quantified with the use of lectins. Lectins are a well-known family of carbohydrate-binding proteins, i.e., macromolecules that are highly specific for given glycans on the basis of their sugar moiety structures and sequences. Lectins can be classified into distinct groups according to their carbohydrate specificity including, but not limited to, fucose-specific, mannose specific, N-acetylglucosamine-specific, and galactose/N-acetylglucosamine- specific lectins. Accordingly, lectins capable of identifying subjects with brain injury may be used in either individually or in any combination thereof.

Figure 1 shows lectins with increase in binding of glycans in saliva samples. The y-axis shows the increase (fold change) in the average of injury samples compared to the average of the uninjured healthy control samples which is represented by the y-axis value of 1.00. As seen in the figure, not only TBI but also orthopedic damage gives rise to increase level of PTL-1 and CNL in saliva, although the increase was significantly lower than in TBI samples.

Figure 2 shows lectins with increase in binding of glycans in urine samples. The y-axis shows the increase (fold change) in the average of injury samples compared to the average of the uninjured healthy control samples which is represented by the y-axis value of 1.00. As seen in the figure, not only TBI but also orthopedic damage gives rise to increase level several glycans in urine, although the increase was significantly lower than in TBI samples. Accordingly, the selection of lectins is essential.

According to one embodiment the method comprises steps of
a) providing urine and/or saliva sample from said subject,
b) determining level of binding of at least one glycan-based biomarker in said urine and/or saliva sample to at least one lectin,
c) comparing the determined level of binding to two control levels of said glycan-based biomarker, wherein said two control levels are
   - the level of binding of said at least one glycan-based biomarker to said at least one lectin in saliva and/or urine of an uninjured subject and
   - the level of binding of said at least one glycan-based biomarker to said at least one lectin in saliva and/or urine of a subject suffering from orthopaedic injury,
d) providing a diagnosis based on said comparing, wherein ≥20% increased level of binding of said at least glycan-based biomarker to said at least one lectin compared to the two control levels is indicative of TBI in said subject wherein
   - for urine sample, the at least one lectin is selected from a group consisting of: UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I, and
   - for saliva sample the at least one lectin is selected from a group consisting of PTL-1 (PTL, WBA-I), CNL, GAL7-S and CSA.

According to one embodiment for urine sample the lectin array comprises one or more lectins selected from a group consisting of GAL1, ORYSATA, PALa, LENTIL, F17AG, LcH (LCA) and DISCOIDIN I.

According to a particular embodiment the method is performed using a lectin array comprising, for urine samples, one of more lectins selected from a group consisting of UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I, and for saliva samples one or more lectins selected from a group consisting of PTL-1 (PTL, WBA-I), CNL, GAL7-S and CSA.

The advantage of the embodiments shown above is that the structure of the glycan-based biomarker does not need to be known. The only requirement is that lectin is specific enough for glycans relevant to TBI, i.e. the diagnosis is not distorted by other pathological conditions.

According to another embodiment the method includes analysis of the glycan-based biomarkers indicative to TBI. The analysis may include treatment of the samples with N-glycosidase and purification through solid-phase extraction. MALDI TOF mass spectrometry is a particular method since it is not prone to fragment the N-glycans.

Relative abundances of neutral-N-glycans and acidic N-glycans in urine samples are shown in figure 3 and 4, respectively, and relative abundances of neutral-N-glycans and acidic N-glycans in saliva samples are shown in figure 5 and 6, respectively as determined by MALDI-TOF mass spectrometry after treatment of the samples with N-glycosidase and purification through solid-phase extraction. The bars show N-glycan structures of increased relative abundance in TBI samples compared to the average of uninjured healthy control samples.

It was found that suitability of the N-glycans as biomarkers of TBI cannot be judged on basis of the highest apparent increase level in body fluid of a subject suffering from TBI vs. N-glycan level in healthy controls merely by comparing the bars in the graphs. This is because not only TBI but also tissue damages such as orthopaedic injuries liberate glycans to body fluids.

Figure 7 shows the abundance of signal of H4N3F3S1 in samples obtained from subjects having TBI, subjects suffering from orthopaedic injury, and subjects not suffering any of those. The signal of H4N3F3S1 was significantly higher in urine samples obtained from subjects suffering from TBI than in urine samples obtained from subjects suffering from orthopaedic injury and healthy controls. In addition, the glycan-based biomarker comprising H4N3F3S1 appeared in urine a few hours after the brain injury, which is essential for an early biomarker test. Accordingly, the N-glycans suitable as marker for TBI have to have good TBI-specificity when the orthopaedic samples are considered as well.

Figure 8 shows the abundance of signal of H5N4F2P1 in saliva samples obtained from subjects having TBI, subjects suffering from orthopaedic injury, and subjects not suffering any of those. The signal of H5N4F2P1 was significantly higher in saliva samples obtained from subjects suffering from TBI than saliva samples of healthy controls. Furthermore, the level of H5N4F2P1 was higher in samples obtained from subjects having TBI than in samples obtained from subjects having orthopaedic injury. In addition, the glycan-based biomarker comprising H5N4F2P1 appeared in saliva a few hours after the brain injury, which is essential for an early biomarker test. Accordingly, the N-glycans suitable as marker for TBI has to have good TBI-specificity when the orthopaedic samples are considered as well.

Pauci-mannose N-glycans, high-mannose N-glycans, and monoantennary sialylated N-glycans represent intracellular glycosylation while being less common on cell surface. Accordingly, their elevated level implies disruption on cells upon injury and release of the "contents" to the surroundings. In other words, glycoproteins or glycopeptides carrying those glycans would be present in elevated amounts after TBI.

The pauci-mannose N-glycan structures share common N-glycan core structure and terminal α-mannose residue. Such structures are rare on the cell surface and are associated with intracellular glycosylation, for example lysosomal glycoproteins.

High-mannose N-glycan structures share in common terminal α1,2-linked mannose residues. Also these structures are rare on the cell surface are associated with intracellular glycosylation, for example endoplasmic reticulum (ER) glycoproteins.

Monoantennary sialylated N-glycan structures share common monoantennary N-glycan core structure and sialylation of the antenna. Also these structures are associated with intracellular glycosylation, for example lysosomal glycoproteins.

Pauci-mannose N-glycans present in elevated amounts in urine after TBI and suitable for the method are H4N2F1 and H3N2 as determined by MALDI-TOF mass spectrometry. Exemplary structure of H4N2F1 (**1**) and H3N2 (**2**) are shown below.

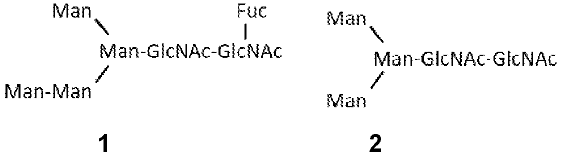

A high-mannose N-glycan present in elevated amount in urine after TBI and suitable for the method is H9N2 as determined by MALDI-TOF mass spectrometry. An exemplary structure of H9N2 (**3**) is shown below.

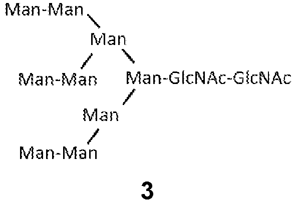

Monoantennary sialylated N-glycans present in elevated amount in urine after TBI are H4N3F1S1 and H4N3F3S1 as determined by MALDI-TOF mass spectrometry. Exemplary structure of H4N3F1S1 (**4**) and H4N3F3S1 (**5**) is shown below.

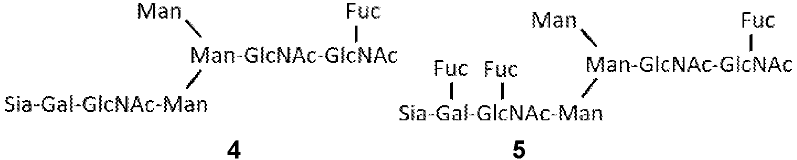

When the body fluid is saliva, the at least one glycan-based biomarker present in elevated amounts after TBI and suitable for the method comprises a glycan-based biomarker comprising a N-glycan selected from a group consisting of H5N4F2P1, H5N4F3P1, H5N5F1S1, and H3N2F1, wherein P is sulphate or phosphate ester, preferably sulphate.

Regarding H5N4F2P1, P refers to one sulfate or phosphate ester, most likely sulfate; F2 refers to two fucose residues, i.e. a core fucose, and an antenna fucose; and H5N4 is a biantennary-size complex-type N-glycan. Accordingly, the N-glycans of formula H5N4F2P1 are biantennary N-glycans with Lewis x or Lewis y on one antenna, and 3'-sulfo-LacNAc (**6**) or 6-sulfo-LacNAc (**7**) on the other antenna.

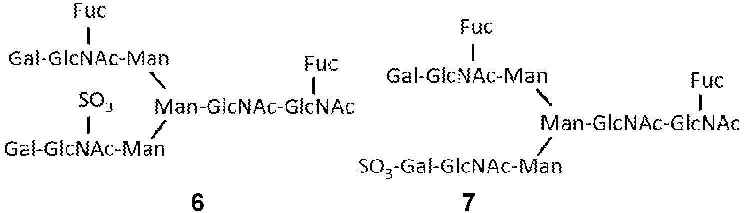

Regarding H5N4F3P1, the formula can be represented by structures (**8-11**).

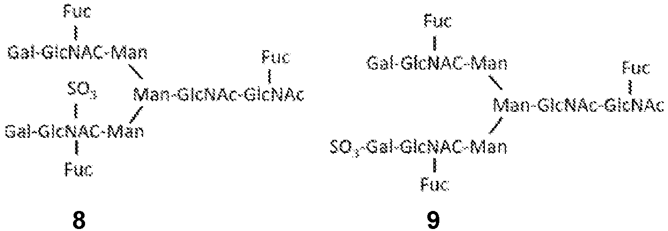

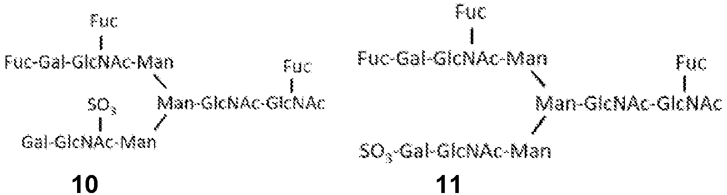

Regarding H5N5F1S1, the N-glycan can be presented as a biantennary-size complex-type N-glycan with proposed bisecting GlcNAc structure (**12**; GlcNAc β1,4-linked to the branching β1,4Man residue).

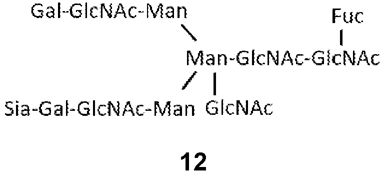

Further N-glycans (**13-16**) also comprising "H5N5" motif and bisecting GlcNAc structure are shown below.

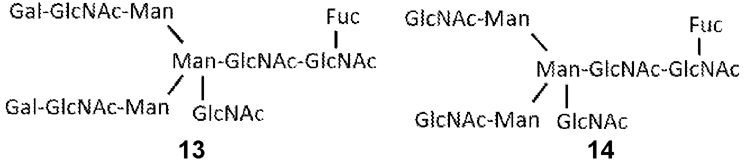

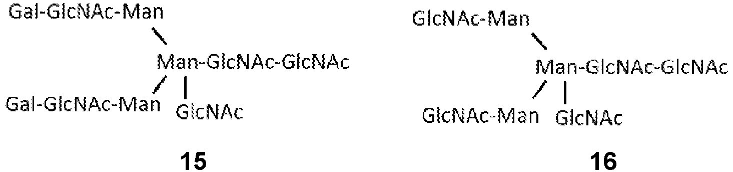

Regarding H3N2F1, the N-glycan is of paucimannose type, and it has four possible isoforms, namely
Manα1-3(Manα1-6)Manβ1-4GlcNAcβ1-4(Fucα1-6)GlcNAc,
Manα1-2Manα1-3Manβ1-4GlcNAcβ1-4(Fucα1-6)GlcNAc,
Manα1-3Manα1-6Manβ1-4GlcNAcβ1-4(Fucα1-6)GlcNAc, and
Manα1-6Manα1-6Manβ1-4GlcNAcβ1-4(Fucα1-6)GlcNAc.

Its exemplary structure (**17**) structure is shown below.

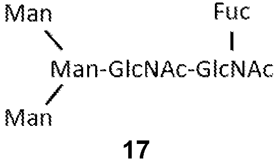

Thus, according to another embodiment the *in vitro* method of diagnosing traumatic brain injury (TBI) of the present invention comprises the steps of:
a) providing a urine and/or a saliva sample obtained from the subject,
b) determining level of at least one glycan-based biomarker in the sample,
c) comparing the determined level to a control level of said glycan-based biomarker, wherein said control level is the level of said biomarker in saliva and/or urine of an uninjured subject, and
d) providing a diagnosis based on said comparing, wherein ≥20% increased level of binding of said at least glycan-based biomarker compared the control level is indicative of TBI in said subject wherein
   - for the urine, the at least one glycan-based biomarker comprises a N-glycan selected from a group consisting of H4N2F1, H3N2, H9N2, H4N3F1S1, and H4N3F3S1, and/or
   - for the saliva sample, the at least one glycan-based biomarker comprises a N-glycan selected from a group consisting of H5N4F2P1, H5N4F3P1, H5N5F1S1, and H3N2F1, wherein
   H is hexose, N is N-acetyl hexosamine, F is fucose, S is sialic acid, and P is sulphate or phosphate ester, preferably sulphate.

The N-glycans can be determined by using one or more of mass spectrometry, lectin binding, antibody binding, aptamer binding.

The level of N-glycan in the sample can be determined e.g. by one or more of: intensity of the MS signal of the N-glycan, level of lectin binding lectin, level of antibody binding, and level of aptamer binding.

When the sample is urine, and the lectin binding is preferably determined using an array comprising one or more lectins selected from a group consisting of UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I, preferably GAL1, ORYSATA, PALa, LENTIL, F17AG, LcH (LCA) and DISCOIDIN I.

When the sample is urine, the lectin binding is preferably determined using an array comprising one or more lectins selected from a group consisting of PTL-1 (PTL, WBA-I), CNL, GAL7-S and CSA.

It was also surprisingly found that the N-glycan profile in saliva and urine differ significantly from each other. This phenomenon can be considered when selecting the most suitable glycans for TBI diagnosis.

Preferable glycan structure groups found to be particularly relevant biomarkers of TBI in urine and saliva are collected in Table 1.

**Table 1.**

| **urine** | |
|---|---|
| 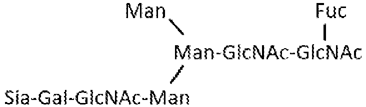 | Acidic N-glycan |
| | Hybrid type, monoantennary sialylated |
| | H4N3F1S1 |
| 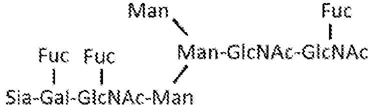 | Acidic N-glycan |
| | Hybrid type, monoantennary sialylated |
| | H4N3F3S1 |
| 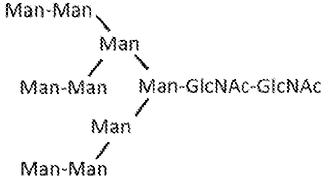 | Neutral N-glycan |
| | High-mannose type |
| | H9N2 |
| 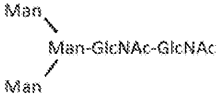 | Neutral N-glycan |
| | Pauci-mannose type |
| | H3N2 |
| 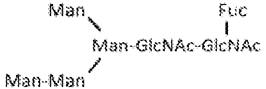 | Neutral N-glycan |
| | Fucosylated pauci-mannose type |
| | H4N2F1 |

| **saliva** | |
|---|---|
| 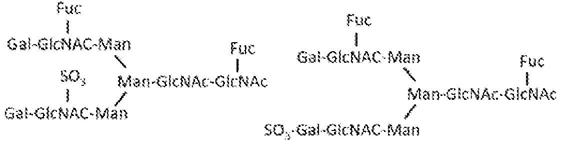 | Acidic N-glycan |
| | Sulphate/phosphate ester |
| | H5N4F2P1 |
| 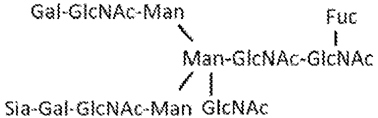 | Acidic N-glycan |
| | Terminal N-acetyl hexosamine, bisecting |
| | GlcNAc structure |
| | H5N5F1S1 |
| 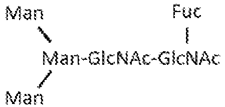 | Neutral N-glycan |
| | Pauci-mannose type, core fucosylated |
| | H3N2F1 |

It is also possible to provide two body fluid samples from the subject, i.e. a saliva sample and a urine sample, determine the level of the at least one glycan-based biomarker in the saliva sample and in the urine sample, and provide the diagnosis based on the determined level of the at least one glycan-based biomarker in the saliva sample and in the urine sample, wherein increased levels of the at least one glycan-based biomarker in the sample and the further sample compared to normal control levels is indicative of TBI. The use of two body fluid samples allows the use of the best TBI biomarkers in saliva and urine for diagnosis. According to an exemplary embodiment H4N3F3S1 is used as a TBI biomarker in urine sample and H5N4F2P1 is used as a biomarker in saliva samples.

The determining of the glycan-based biomarkers comprising the N-glycans from body fluid can be done by any method known in the art. Exemplary non-limiting method are mass spectrometry, chromatography and separation methods, affinity binding like lectin binding, antibody binding and aptamer binding, or gel electrophoresis.

According to an exemplary embodiment the determining comprises the following steps
i) separating the at least one glycan-based biomarker comprising N-glycan from the sample,
ii) treating the separated glycan-based biomarker with N-glycosidase, to release the N-glycan from the glycan-based biomarker, and
iii) determining level of the N-glycan in the body fluid sample.

According to an exemplary embodiment the separating comprises treating the sample with an alcohol such as ethanol to produce a precipitate comprising the glycan-based biomarker. Treatment of the precipitate with N-glycosidase such as N-glycosidase F enzyme liberates the N-glycan of the glycan-based biomarker and other oligosaccharides present in the sample. The oligosaccharides are preferably purified prior to mass spectrometric analysis. The strength of the MS signal of the N-glycan is compared to a control level. A level higher than the control level, such as at least 1.5 times higher, is an indication of TBI in the subject.

According to another embodiment the determining performed by using specific glycan binders such as lectins, or antibodies raised against the glycan structure or the protein structure of the glycan-based biomarker. Alternatively, a binder element can be a molecularly printed polymer (MIP) to the glycan or protein epitope of the glycan-based biomarker. The binder can also be an aptamer which is an oligonucleotide or peptide that specifically binds the target molecule.

Standard techniques of protein microarray technology can be applied to analyze the glycan-based biomarkers. In such microarrays, lectins are immobilized on a solid support, such as a slide, in a high spatial density. Each lectin may be arrayed at several concentrations and in replicates on each slide. The concentration ranges may be tailored for each of the lectins and calibrated to provide a linear response within the same range, regardless of the affinity of the lectin. A sample of intact glycan-based biomarkers is applied to the array, and its binding pattern is detected by a label, such as a fluorescent label, a radioactive label, or a chemiluminescent label, which is placed either on the biomarker itself or on the lectin directed toward the carbohydrate moieties of the biomarker. Streptavidin may be used for detecting biotinylated samples. Also, sandwich-based methods which utilize antibody detection may be employed, as is apparent to those with ordinary skill in the art.

Suitable microarray substrates include, but are not limited to, glass, silica, aluminosilicates, borosilicates, metal oxides such as alumina and nickel oxide, gold, various clays, nitrocellulose, or nylon. In some embodiments a glass substrate is preferred. In other embodiments, the substrate may be coated with a compound to enhance binding of the lectin to the substrate. In some further embodiments, lectins have been arrayed on a nitrocellulose membrane- coated glass slide or plastic backing plate. In some still further embodiments, one or more control lectins are also attached to the substrate.

In some embodiments, a commercially available lectin array, which encompasses one standard glass slide, which is spotted with 8 wells of identical lectin arrays, may be employed. Each lectin, together with the positive controls is arrayed in duplicate. The slide comes with an 8-well removable gasket which allows for the process of 8 samples using one slide. Four-slide slides can be nested into a tray, which matches a standard microplate and allows for automated robotic high throughput process of 64 arrays simultaneously. Unlike other conventional methods, e.g., liquid chromatography and mass spectrometry, lectin microarrays enable rapid and high-sensitivity profiling of complex glycan features without the need for liberation of glycans. Target samples include an extensive range of glycoconjugates involved in cells, tissues, body fluids, as well as synthetic glycans and their mimics. Various procedures for rapid differential glycan profiling have been developed for glycan-related biomarkers and are commercially available.

When the sample is urine, the array comprises preferably one or more lectins selected from a group consisting of UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I. According to another embodiment the lectins are selected from a group consisting of GAL1, ORYSATA, PALa, LENTIL, F17AG, LcH (LCA) and DISCOIDIN I.

When the sample is saliva, and the array comprises one or more lectins selected from a group consisting of PTL-1 (PTL, WBA-I), CNL, GAL7-S and CSA.

According to one embodiment the method comprises performing at least one neuroimaging procedure selected from a group consisting of x-ray, computerized tomography, and magnetic resonance imaging on the subject. The neuroimaging procedure is preferably performed only if the level of the at least one glycan-based biomarker in body fluid subject is indicative of TBI.

The present invention also concerns use of at least one glycan-based biomarker comprising N-glycan selected from H4N2F1, H3N2, H9N2, H4N3F1S1, H4N3F3S1, wherein H is hexose, N is N-acetyl hexosamine, F is fucose, S is sialic acid, as an indicator of traumatic brain injury in urine, wherein the structures of the N-glycans are disclosed in table 1.

The present invention also concerns use of at least one glycan-based biomarker comprising N-glycan comprising N-glycan selected from a group consisting of H5N4F2P1, H5N4F3P1, H5N5F1S1, and H3N2F1, wherein H is hexose, N is N-acetyl hexosamine, F is fucose, S is sialic acid, and P is sulphate or phosphate ester, preferably sulphate, as a biomarker of traumatic brain injury in saliva, wherein the structures of the N-glycans are disclosed in table 1.

Disclosed is also a new kit or a device for use in the method. The kit or the device comprises at least one lectin, antibody, or combination thereof that selectively binds to a glycan-based biomarker comprising a N-glycan, and a control for comparing to a measured value of binding wherein the N-glycan- is selected from a group consisting of H4N2F1, H3N2, H9N2, H4N3F1S1, and H4N3F3S1 H5N4F2P1, H5N4F3P1, H5N5F1S1, and H3N2F1 wherein H is hexose, N is N-acetyl hexosamine, F is fucose, and S is sialic acid and P is sulphate or phosphate ester. The at least one lectin of the kit and the device is preferably selected from a group consisting of UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA), DISCOIDIN I, PTL-1, CNL, GAL7-S and CSA

The kit can also comprise a washing solution or instructions for making a washing solution, in which the combination of the capture reagents and the washing solution allows capture of the biomarkers on the solid support or column for subsequent detection by, e.g., antibodies or mass spectrometry. In a further embodiment, a kit can comprise instructions for suitable operational parameters in the form of a label or separate insert. For example, the instructions may inform a healthcare professional or consumer about how to collect the sample, how to wash the probe or the particular biomarkers to be detected, etc. In yet another embodiment, the kit can comprise one or more containers with biomarker samples, to be used as standard(s) for calibration.

As is apparent to a skilled person, the present lectin array kit can be used with either a label-based method or as a sandwich-based method. In one embodiment, the label-based method is used for biotinylated samples containing proteoglycans and glycoproteins for direct detection on the array via a Cy3 equivalent dye-conjugated Biotin-Streptavidin complex. In another embodiment, a sandwich-based method is used for antibody detection of glycocalyx elements (glycolipids, glycoproteins, etc.) captured on the array. Labelled reporter antibodies specific for the glycocalyx elements of interest may be provided in the kit or supplied by the user of the kit. An example protocol for this procedure with a general "Antibody Cocktail" may be included in a user manual. In some non-limiting embodiments, specific antibody concentrations and conditions may need to be determined by the end user.

In one embodiment of the biomarker detection kit, HRP protein or other enzymes and fluorescent light or change in absorbance may be employed in order to detect the biomarker in a body fluid and to indicate the quantity of the biomarker in percentage. In one embodiment of the biomarker detection kit, colloidal gold, gold particles, latex particles or similar colloids or particles may be employed in order to detect the quantity of the biomarker. These enable visual detection without instrumentation. Any of the above may be incorporated into a portable application that indicates the severity of brain damage on a scale comprising, but not limited to, none, mild, moderate, and severe. In another embodiment, an analogous yes/no reply is received. These examples do not exclude other possible embodiments.

In some embodiments, the present disclosure provides use of at least one antibody in a kit or in a device to detect brain damage, where the antibody may be a polyclonal or a monoclonal antibody of any species, or a fragment thereof, either enzymatically cleaved or recombinantly produced, or a humanized antibody, and where the antibody recognizes and binds glycan, glycoprotein, peptidoglycan, proteoglycan, glycolipid, protein, small molecule, lectin, or antibody of another species (generally 'antigens'). Said antibody may be used, for instance, as
i) a capture reagent, wherein the antibody is immobilized on a solid substrate to bind its antigen from a sample medium;
ii) an antibody that is immobilized on a solid substrate to bind an analyte-specific capture reagent (for example lectin) so that the bound agent (lectin) is able to capture the analyte (glycan) from a sample;
iii) a primary detection reagent, wherein an antibody conjugated to any label (labelled antibody) recognizes and directly binds an antigen;
iv) a secondary detection reagent, wherein a labelled antibody recognizes and binds a primary detection reagent that is bound to the analyte. For example, a labelled antibody binds to a lectin that has bound to its cognate glycan, or a labelled antibody from one species (e.g. goat) that recognizes and binds an antibody of another species (e.g. mouse) which has bound its antigen;
v) an antibody for recognizing and binding a non-glycan part of a glycan-containing molecule, e.g. a glycoprotein, where the glycoprotein or a fragment thereof is first bound to e.g. lectin via its glycan moiety and then is recognized and bound by an antibody that is specific to the peptide part of the molecule; or
vi) antibody for use in immunoblotting assays.

The kit may also comprise a combination of antibodies for different purposes.

All embodiments, details, advantages, and the like of the present kit also apply to a device for use in different aspects and embodiments of the present disclosure.

Also, all embodiments, details, advantages, and the like of the present methods apply to the present kit, and vice versa. In particular, one or more compounds, compositions, or reagents disclosed as suitable for carrying out the present methods may be comprised in the present kit. Likewise, anything disclosed with reference to the kit, apply to the present methods as well.

### EXPERIMENTAL

A clinical study collecting body flid samples from injured patients and uninjured healthy control subjects was carried out. Urine and saliva were collected from 24 patients diagnosed with mild traumatic brain injury (concussion), from 16 patients with an orthopaedic injury (fracture in leg or arm/hand, but no head injury) and from 29 uninjured healthy control subjects who did not have TBI nor fractures during the past year. Upon collection, samples were frozen and stored at -70 °C until analysed.

Urine and saliva samples were analysed by using either chromatographic purification followed by analysis by Mass Spectrometry (MALDI-TOF), or lectin array analysis.

### Mass Spectrometry

The structural data was acquired by mass spectrometry (MS, MALDI-TOF). The principles for sample preparation, data collection and data analysis were same for all body fluids, and it's briefly summarized as follows.

The samples were pre-treated with ethanol precipitation and the ethanol precipitate was subjected to N-glycan detachment from sample glycoproteins with N-glycosidase F enzyme. Liberated oligosaccharides were purified by solid-phase extraction (SPE) in 96-well plate format: passing through C18 silica in water and then purification by Hypercarb graphitized carbon. The neutral and acidic N-glycan fractions were eluted separately. The two N-glycan fractions were then separately analyzed by MALDI-TOF mass spectrometric profiling using a Bruker Ultraflex III TOF/TOF instrument.

The spectra were compared to databases/libraries of glycan structures. The glycan structures represented by the peaks (m/z ratio) of the spectra were deduced based on this data analysis and knowledge of human glycan structures and synthesis pathways. Relative abundances of the glycans in a sample were calculated based on the area or height of the peak.

### Lectin arrays

Lectins spotted on a glass slide captured the glycan biomarkers from the sample. The immobilized biomarkers were detected by fluorescence visualization of the array.

The lectin-binding results showed a statistically significant increased binding of specific glycans, relative to uninjured healthy samples, both in saliva and urine following a head injury.

In saliva, four lectins showed an increase of ≥70% with statistical significance level of p ≤0.05 in TBI samples compared to healthy samples, and furthermore, with none or only minimal increase in orthopaedic samples, rendering TBI vs. Orthopaedic ratio of ≥60% at statistical significance level of p ≤0.1 (Figure 1, Table 2).

In urine, 20 lectins showed an increase of ≥20% with statistical significance level of p ≤0.05 in TBI samples compared to healthy samples, and furthermore, with none or only minimal increase in orthopaedic samples, rendering TBI vs. Orthopaedic ratio of ≥20% at statistical significance level of p ≤0.05 (Figure 2, Table 3).

**Table 2 Lectins with ≥70% increase in binding of glycans at statistical significance of p ≤0.05 in TBI-samples vs. uninjured healthy control samples (HC), and ≥60% difference in TBI samples vs. orthopaedic samples (Ortho) at p ≤0.1. The data compares average values of TBI samples (n = 24), orthopaedic samples (n = 16), and uninjured healthy samples (n = 29). The table shows results for saliva samples.**

| **Lectin** | **Full name or Origin** | **Fold Change** | | |
|---|---|---|---|---|
| | | **TBI/HC** | **Ortho/HC** | **TBI/Ortho** |
| PTL-1 (PTL, WBA-I) | Psophocarpus | 2.04 | 1.10 | 1.85 |
| CNL | Clitocybe nebularis lectin | 1.95 | 1.22 | 1.60 |
| GAL7-S | Human galectin7-S lectin | 1.76 | 0.73 | 2.42 |
| CSA | Cytisus scoparius lectin | 1.71 | 0.90 | 1.89 |

**Table 3 Lectins with ≥20% increase in binding of glycans at statistical significance of p ≤0.05 in TBI-samples vs. uninjured healthy control samples (HC), and ≥20% difference in TBI samples vs. orthopaedic samples (Ortho) at p ≤0.05. The data compares average values of TBI samples (n = 24), orthopaedic samples (n = 16), and uninjured healthy samples (n = 29). The table shows results for urine samples.**

| **Lectin** | **Full Name or Origin** | **Fold Change** | | |
|---|---|---|---|---|
| | | **TBI/HC** | **Ortho/HC** | **TBI/Ortho** |
| UDA | Urtica dioica | 2.15 | 1.05 | 2.06 |
| GRFT | Griffithia sp. Lectin | 2.15 | 1.11 | 1.93 |
| CALSEPA | Calystegia sepium lectin | 2.12 | 1.27 | 1.67 |
| BANLEC | Musa acuminata lectin | 2.05 | 0.94 | 2.19 |
| NPA (NPL, DL) | Narcissus pseudo narcissus | 1.89 | 1.24 | 1.53 |
| HHA (HHL, AL) | Hippeastrum hybrid | 1.87 | 1.21 | 1.55 |
| Con A | Concanavalin A | 1.77 | 0.87 | 2.03 |
| GAL1 | Human galectin1 lectin (stable form) | 1.67 | 0.68 | 2.44 |
| BC2L-A | Burkholderia cenocepacia lectin | 1.63 | 0.93 | 1.74 |
| SAMB | Sambucus Sieboldiana Lectin | 1.57 | 1.01 | 1.56 |
| ORYSATA | Oryza sative lectin | 1.55 | 1.09 | 1.43 |
| PALa | Phlebodium aureum lectin | 1.52 | 0.98 | 1.55 |
| PTL-I (PTL, WBA-I) | Psophocarpus | 1.51 | 0.91 | 1.66 |
| GS-I (GSL-I, BSL-I) | Griffonia (Bandeiraea ) simplicifolia I | 1.42 | 1.01 | 1.41 |
| PSA (PEA) | Pisum sativum | 1.42 | 0.88 | 1.61 |
| LENTIL | Lentil lectin | 1.40 | 0.75 | 1.86 |
| LEA (LEL, TL) | Lycopersicon esculentum | 1.38 | 0.75 | 1.84 |
| F17AG | E. coli lectin | 1.29 | 0.97 | 1.33 |
| LcH (LCA) | Lens culinaris | 1.28 | 0.95 | 1.35 |
| DISCOIDIN I | Dictyostelium discoideum lectin | 1.22 | 1.00 | 1.22 |

## Claims

1. An *in vitro* method of diagnosing traumatic brain injury (TBI) in a subject, the method comprising the steps of:
a) providing urine and/or saliva sample from said subject,
b) determining level of binding of at least one glycan-based biomarker in said urine and/or saliva sample to at least one lectin,
c) comparing the determined level of binding to two control levels of said glycan-based biomarker, wherein said two control levels are
- the level of binding of said at least one glycan-based biomarker to said at least one lectin in saliva and/or urine of an uninjured subject and
- the level of binding of said at least one glycan-based biomarker to said at least one lectin in saliva and/or urine of a subject suffering from orthopaedic injury, and
d) providing a diagnosis based on said comparing, wherein ≥20% increased level of binding of said at least glycan-based biomarker to said at least one lectin compared to the two control levels is indicative of TBI in said subject wherein
- for urine sample, the at least one lectin is selected from a group consisting of: UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I, and
- for saliva, sample the at least one lectin is selected from a group consisting of PTL-1 (PTL, WBA-I), CNL, GAL7-S and CSA.

2. The method according to claim 1 wherein for urine sample the at least one lectin is selected from a group consisting of GAL1, ORYSATA, PALa, LENTIL, F17AG, LcH (LCA) and DISCOIDIN I.

3. An *in vitro* method of diagnosing traumatic brain injury (TBI) in a subject, the method comprising the steps of:
a) providing a urine and/or a saliva sample obtained from the subject,
b) determining level of at least one glycan-based biomarker in said urine and/or saliva sample,
c) comparing the determined level to a control level of said glycan-based biomarker, wherein said control level is level of said biomarker in saliva and/or urine of an uninjured subject and
d) providing a diagnosis based on said comparing, wherein ≥20% increased level of compared the control level is indicative of TBI in said subject wherein
- for the urine sample, the at least one glycan-based biomarker comprises a N-glycan selected from a group consisting of H4N2F1, H3N2, H9N2, H4N3F1S1, and H4N3F3S1, and/or
- for the saliva sample, the at least one glycan-based biomarker comprises a N-glycan selected from a group consisting of H5N4F2P1, H5N4F3P1, H5N5F1S1, and H3N2F1, wherein
- wherein
H4N2F1 is 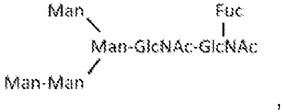
H3N2 is 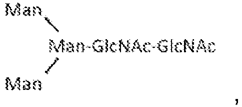
H9N2 is 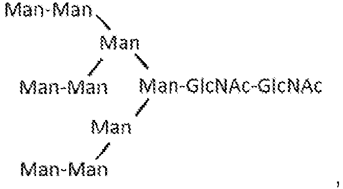
H4N3F1S1 is 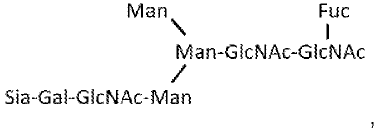
H4N3F3S1 is 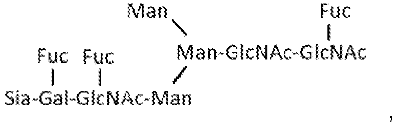
H5N4F2P1 is selected from 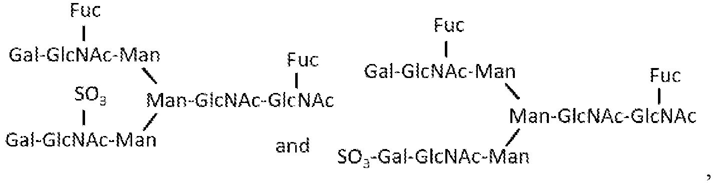
H5N5F1S1 is 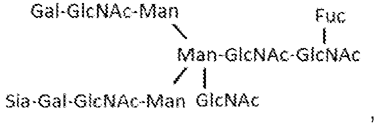
H3N2F1 is 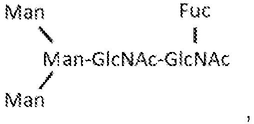 and
H5N4F3P1 is selected from a group consisting of

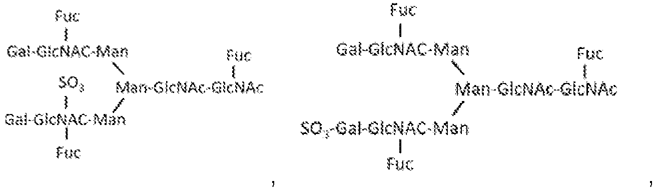

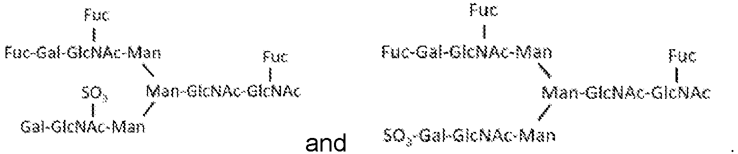

4. The method according to claim 3 wherein the glycan-based biomarker is a glycoprotein or a cleavage product or a biodegradation product thereof.

5. The method according to claim 3 wherein the determining of step b) comprises
i) separating the least one glycan-based biomarker from the sample,
ii) treating the separated glycan-based biomarker with N-glycosidase to provide the N-glycan, and
iii) determining level of the N-glycan of the glycan-based biomarker by mass spectrometry.

6. The method according to claim 3 wherein the level of the at least one glycan-based biomarker in said urine and/or saliva sample and said control level is determined by using one or more of mass spectrometry, lectin binding, antibody binding, aptamer binding.

7. The method according to claim 5 wherein the sample is urine, and the lectin binding is determined using an array comprising one or more lectins selected from a group consisting of UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) and DISCOIDIN I, preferably GAL1, ORYSATA, PALa, LENTIL, F17AG, LcH(LCA) and DISCOIDIN I.

8. The method according to claim 5 wherein the sample is saliva, and the lectin binding is determined using an array comprising one or more lectins selected from a group consisting of PTL-1 (PTL, WBA-I), CNL, GAL7-S and CSA.

9. The method according to any one of claims 1 to 8 wherein the subject is a child.

10. The method according to any one of claims 1 to 9 comprising performing at least one neuroimaging procedure selected from a group consisting of x-ray, computerized tomography, and magnetic resonance imaging on a subject when level of said at least one glycan-based biomarker in body fluid sample of said subject is indicative of TBI.

11. Use of at least one glycan-based biomarker comprising N-glycan selected from H4N2F1, H3N2, H9N2, H4N3F1S1, and H4N3F3S1, wherein
H4N2F1 is 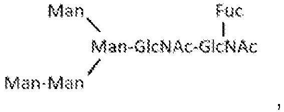
H3N2 is 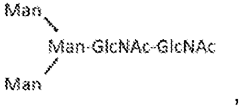
H9N2 is 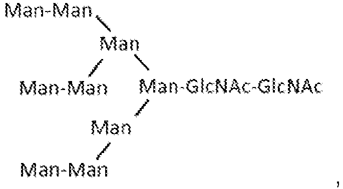
H4N3F1S1 is 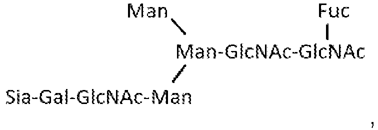 and
H4N3F3S1 is 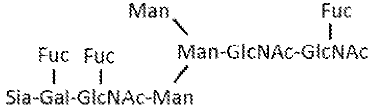 as an indicator of traumatic brain injury in urine.

12. Use of at least one glycan-based biomarker comprising N-glycan selected from a group consisting of H5N4F2P1, H5N4F3P1, HSN5F1S1, and H3N2F1, wherein
H5N4F2P1 is selected from 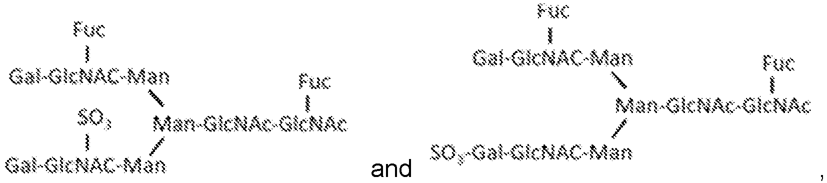
H5N4F3P1 is selected from 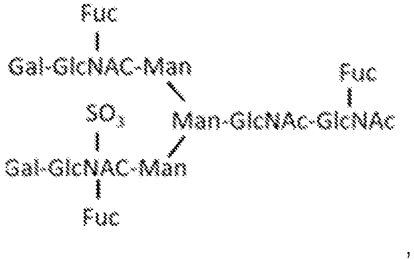 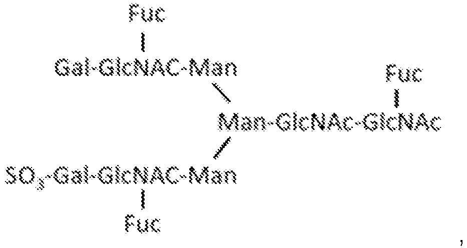 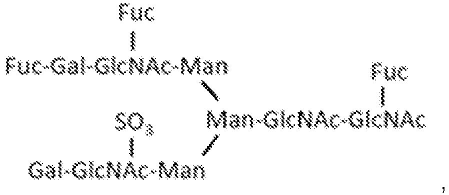 and 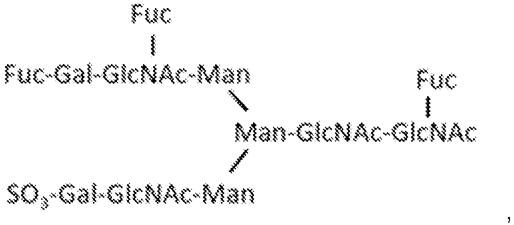
H5N5F1S1 is 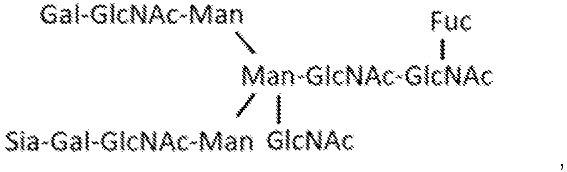 and
H3N2F1 is selected from the group consisting of
Manα1-3(Manα1-6)Manβ1-4GlcNAcβ1-4(Fucα1-6)GlcNAc,
Manα1-2Manα1-3Manβ1-4GlcNAcβ1-4(Fucα1-6)GlcNAc,
Manα1-3Manα1-6Manβ1-4GlcNAcβ1-4(Fucα1-6)GlcNAc, and
Manα1-6Manα1-6Manβ1-4GlcNAcβ1-4(Fucα1-6)GlcNAc, preferably 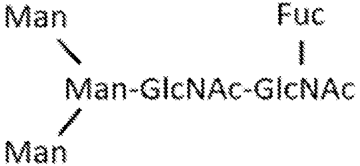 as an indicator of traumatic brain injury in saliva.

## Patentansprüche

1. In-Vitro-Verfahren zum Diagnostizieren einer traumatischen Hirnverletzung (TBI) bei einem Subjekt, wobei das Verfahren die Schritte umfasst:
a) Bereitstellen einer Urin- und/oder Speichelprobe des Subjekts,
b) Bestimmen des Bindungsgehaltes mindestens eines glykanbasierten Biomarkers in der Urin- und/oder Speichelprobe an mindestens ein Lektin,
c) Vergleichen des bestimmten Bindungsgehaltes mit zwei Kontrollgehalten des glykanbasierten Biomarkers, wobei die zwei Kontrollgehalte sind:
- der Bindungsgehalt des mindestens einen glykanbasierten Biomarkers an das mindestens eine Lektin im Speichel und/oder Urin eines unverletzten Subjekts und
- der Bindungsgehalt des mindestens einen glykanbasierten Biomarkers an das mindestens eine Lektin im Speichel und/oder Urin eines Subjekts, das an einer orthopädischen Verletzung leidet,
d) Bereitstellen einer Diagnose auf der Basis des Vergleichens, wobei ein um ≥20 % erhöhter Bindungsgehalt des mindestens einen glykanbasierten Biomarkers an das mindestens eine Lektin im Vergleich zu den beiden Kontrollwerten auf eine TBI bei dem Subjekt hinweist, wobei
- für die Urinprobe das mindestens eine Lektin aus einer Gruppe ausgewählt ist, die besteht aus: UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) und DISCOIDIN I, und
- für die Speichelprobe das mindestens eine Lektin aus einer Gruppe ausgewählt ist, die aus PTL-1 (PTL, WBA-I), CNL, GAL7-S und CSA besteht.

2. Verfahren nach Anspruch 1, wobei für die Urinprobe das mindestens eine Lektin aus einer Gruppe ausgewählt ist, die aus GAL1, ORYSATA, PALa, LENTIL, F17AG, LcH (LCA) und DISCOIDIN I besteht.

3. In-Vitro-Verfahren zum Diagnostizieren einer traumatischen Hirnverletzung (TBI) bei einem Subjekt, wobei das Verfahren die Schritte umfasst:
a) Bereitstellen einer Urin- und/oder Speichelprobe, die von dem Subjekt erhalten wurde,
b) Bestimmen des Gehalts mindestens eines glykanbasierten Biomarkers in der Urin- und/oder Speichelprobe,
c) Vergleichen des bestimmten Gehalts mit einem Kontrollgehalt des glykanbasierten Biomarkers, wobei der Kontrollgehalt der Gehalt des Biomarkers im Speichel und/oder Urin eines unverletzten Subjekts ist, und
d) Bereitstellen einer Diagnose auf der Basis des Vergleichens, wobei ein um ≥20 % erhöhter Gehalt im Vergleich zu dem Kontrollgehalt auf eine TBI bei dem Subjekt hinweist, wobei
- für die Urinprobe der mindestens eine glykanbasierte Biomarker ein N-Glykan umfasst, das aus einer Gruppe ausgewählt ist, die aus H4N2F1, H3N2, H9N2, H4N3F1S1 und H4N3F3S1 besteht, und/oder
- für die Speichelprobe der mindestens eine glykanbasierte Biomarker ein N-Glykan umfasst, das aus einer Gruppe ausgewählt ist, die aus H5N4F2P1, H5N4F3P1, H5N5F1S1 und H3N2F1 besteht, wobei
- wobei
H4N2F1 ist 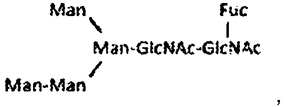
H3N2 ist 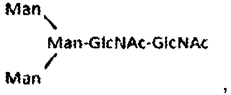
H9N2 ist 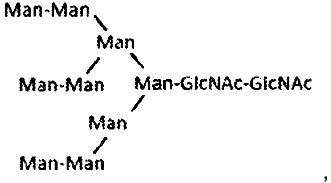
H4N3F1S1 ist 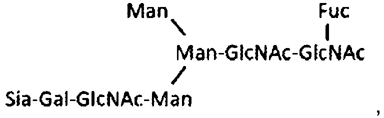
H4N3F3S1 ist 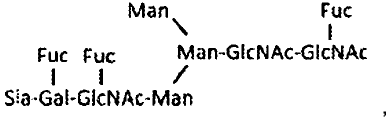
H5N4F2P1 ausgewählt ist aus 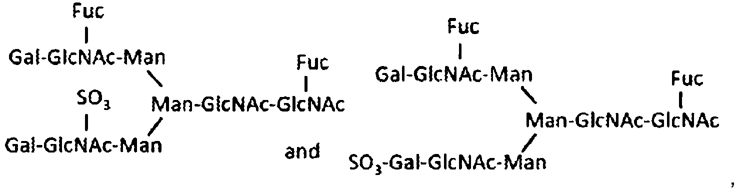
H5N5F1S1 ist 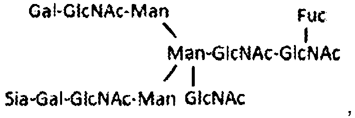
H3N2F1 ist 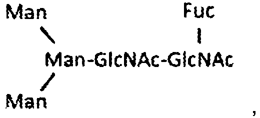 und
H5N4F3P1 aus der Gruppe ausgewählt ist, bestehend aus

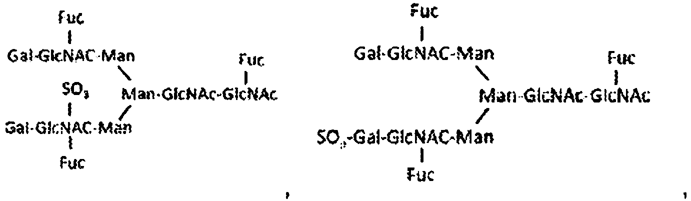

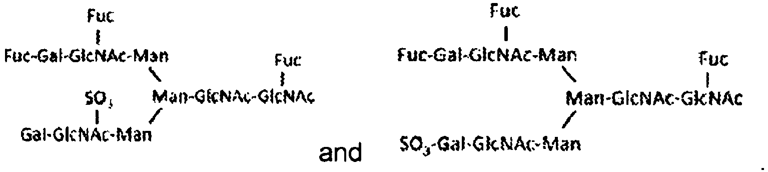

4. Verfahren nach Anspruch 3, wobei der glykanbasierte Biomarker ein Glykoprotein oder ein Spaltprodukt oder ein biologisches Abbauprodukt davon ist.

5. Verfahren nach Anspruch 3, wobei das Bestimmen von Schritt b) umfasst
i) Abtrennen des mindestens einen glykanbasierten Biomarkers von der Probe,
ii) Behandeln des abgetrennten glykanbasierten Biomarkers mit N-Glycosidase, um das N-Glykan bereitzustellen, und
iii) Bestimmen des Gehalts des N-Glykans des glykanbasierten Biomarkers durch Massenspektrometrie.

6. Verfahren nach Anspruch 3, wobei der Gehalt des mindestens einen glykanbasierten Biomarkers in der Urin- und/oder Speichelprobe und der Kontrollgehalt unter Verwendung eines oder mehrerer von Massenspektrometrie, Lektinbindung, Antikörperbindung, Aptamerbindung bestimmt wird.

7. Verfahren nach Anspruch 5, wobei die Probe Urin ist und die Lektinbindung unter Verwendung eines Arrays bestimmt wird, das ein oder mehrere Lektine umfasst, die aus einer Gruppe ausgewählt sind, die aus UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) und DISCOIDIN I, bevorzugt GAL1, ORYSATA, PALa, LENTIL, F17AG, LcH (LCA) und DISCOIDIN I besteht.

8. Verfahren nach Anspruch 5, wobei die Probe Speichel ist und die Lektinbindung unter Verwendung eines Arrays bestimmt wird, das ein oder mehrere Lektine umfasst, die aus einer Gruppe ausgewählt sind, die aus PTL-1 (PTL, WBA-I), CNL, GAL7-S und CSA besteht.

9. Verfahren nach einem der Ansprüche 1 bis 8, wobei das Subjekt ein Kind ist.

10. Verfahren nach einem der Ansprüche 1 bis 9, umfassend das Durchführen mindestens eines Neurobildgebungsverfahrens, das aus einer Gruppe ausgewählt ist, die aus Röntgen, Computertomographie und Magnetresonanzbildgebung bei einem Subjekt besteht, wenn der Gehalt des mindestens einen glykanbasierten Biomarkers in einer Körperflüssigkeitsprobe des Subjekts auf eine TBI hinweist.

11. Verwendung von mindestens einem glykanbasierten Biomarker, der N-Glykan umfasst, das aus H4N2F1, H3N2, H9N2, H4N3F1S1 und H4N3F3S1 ausgewählt ist, wobei
H4N2F1 ist 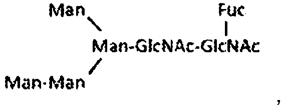
H3N2 ist 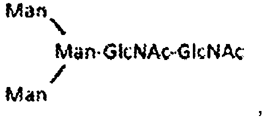
H9N2 ist 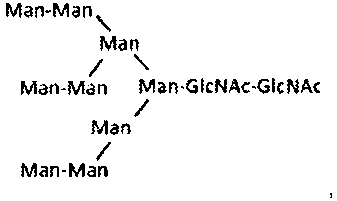
H4N3F1S1 ist 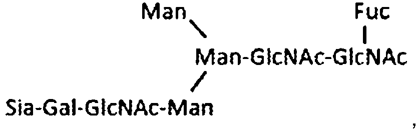 und
H4N3F3S1 ist 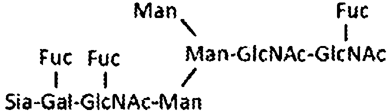
als ein Indikator für eine traumatische Hirnverletzung in Urin.

12. Verwendung von mindestens einem glykanbasierten Biomarker, der N-Glykan umfasst, das aus einer Gruppe ausgewählt ist, die aus H5N4F2P1, H5N4F3P1, H5N5F1S1 und H3N2F1 besteht, wobei
H5N4F2P1 ausgewählt ist aus 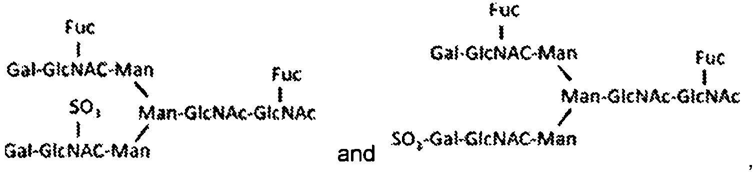
H5N4F3P1 ausgewählt ist aus 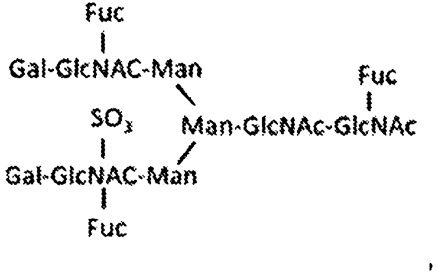 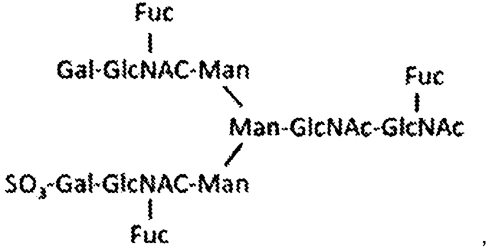 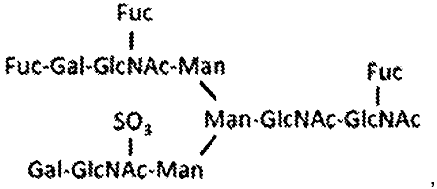 und 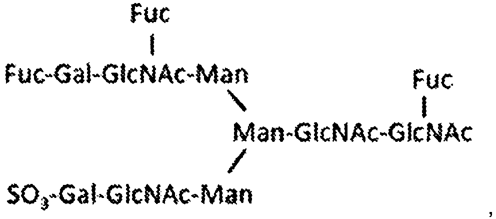
H5N5F1S1 ist 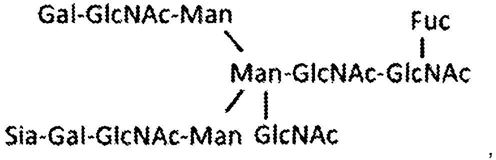 und
H3N2F1 aus der Gruppe ausgewählt ist, bestehend aus
Manα1-3(Manα1-6)Manβ1-4GlcNAcβ1-4(Fucα1-δ)GlcNAc,
Manα1-2Manα1-3Manβ1-4GlcNAcβ1-4(Fucα1-6)GlcNAc,
Manα1-3Manα1-6Manβ1-4GlcNAcβ1-4(Fucα1-6)GlcNAc, und
Manα1-6Manα1-6Manβ1-4GlcNAcβ1-4(Fucα1-6)GlcNAc, bevorzugt 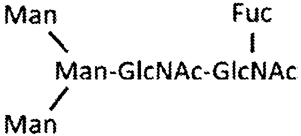
als ein Indikator für eine traumatische Hirnverletzung in Speichel.

## Revendications

1. Procédé *in vitro* de diagnostic d'un traumatisme crânien (TBI) chez un sujet, le procédé comprenant les étapes suivantes :
a) la fourniture d'un échantillon d'urine et/ou de salive dudit sujet,
b) la détermination d'un niveau de liaison d'au moins un biomarqueur à base de glycane dans ledit échantillon d'urine et/ou de salive à au moins une lectine,
c) la comparaison du niveau de liaison déterminé à deux niveaux témoins dudit biomarqueur à base de glycane, dans lequel lesdits deux niveaux témoins sont
- le niveau de liaison dudit au moins un biomarqueur à base de glycane à ladite au moins une lectine dans la salive et/ou l'urine d'un sujet indemne et
- le niveau de liaison dudit au moins un biomarqueur à base de glycane à ladite au moins une lectine dans la salive et/ou l'urine d'un sujet souffrant d'une blessure orthopédique, et
d) l'établissement d'un diagnostic sur la base de ladite comparaison, dans lequel un niveau de liaison accru de 20 % ou plus dudit au moins un biomarqueur à base de glycane à ladite au moins une lectine par rapport aux deux niveaux témoins est indicatif d'un TBI chez ledit sujet, dans lequel
- pour l'échantillon d'urine, la au moins une lectine est choisie dans un groupe constitué de : UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) et DISCOIDIN I, et
- pour l'échantillon de salive, la au moins une lectine est choisie dans un groupe constitué de PTL-1 (PTL, WBA-I), CNL, GAL7-S et CSA.

2. Procédé selon la revendication 1, dans lequel, pour l'échantillon d'urine, la au moins une lectine est choisie dans un groupe constitué de GAL1, ORYSATA, PALa, LENTIL, F17AG, LcH (LCA) et DISCOIDIN I.

3. Procédé *in vitro* de diagnostic d'un traumatisme crânien (TBI) chez un sujet, le procédé comprenant les étapes suivantes :
a) la fourniture d'un échantillon d'urine et/ou de salive obtenu auprès du sujet,
b) la détermination du niveau d'au moins un biomarqueur à base de glycane dans ledit échantillon d'urine et/ou de salive,
c) la comparaison du niveau déterminé à un niveau témoin dudit biomarqueur à base de glycane, dans lequel ledit niveau témoin est un niveau dudit biomarqueur dans la salive et/ou l'urine d'un sujet indemne et
d) l'établissement d'un diagnostic sur la base de ladite comparaison, dans lequel un niveau accru de 20 % ou plus par rapport au niveau témoin indique un TBI chez ledit sujet, dans lequel
- pour l'échantillon d'urine, le au moins un biomarqueur à base de glycane comprend un N-glycane choisi dans un groupe constitué de H4N2F1, H3N2, H9N2, H4N3F1S1 et H4N3F3S1, et/ou
- pour l'échantillon de salive, le au moins un biomarqueur à base de glycane comprend un N-glycane choisi dans un groupe constitué de H5N4F2P1, H5N4F3P1, H5N5F151 et H3N2F1, dans lequel
- dans lequel
H4N2F1 est 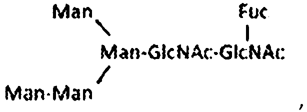
H3N2 est 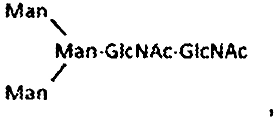
H9N2 est 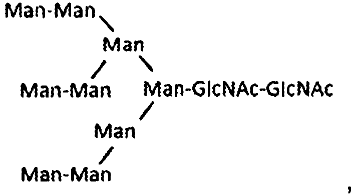
H4N3F1S1 est 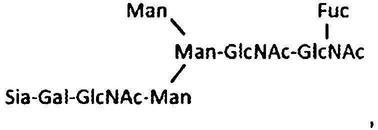
H4N3F3S1 est 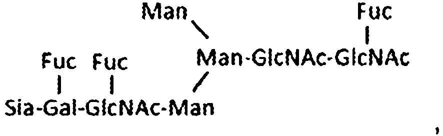
H5N4F2P1 est choisi parmi 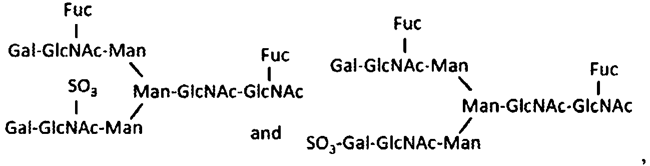
H5N5F1S1 est 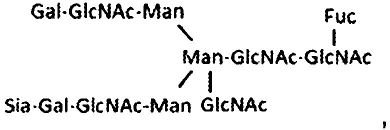
H3N2F1 est 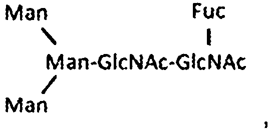 et
H5N4F3P1 est choisi dans un groupe constitué de

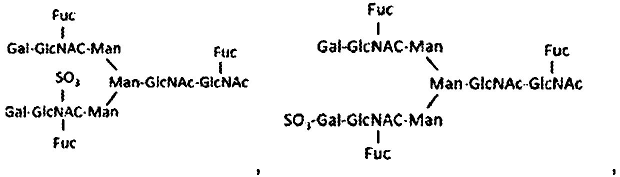

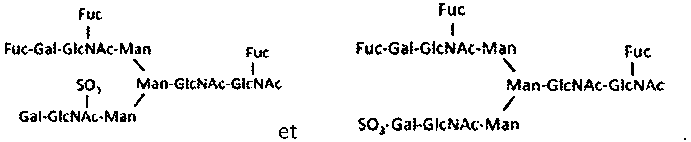

4. Procédé selon la revendication 3, dans lequel le biomarqueur à base de glycane est une glycoprotéine ou un produit de clivage ou un produit de biodégradation de celle-ci.

5. Procédé selon la revendication 3, dans lequel la détermination à l'étape b) comprend
i) la séparation du au moins un biomarqueur à base de glycane de l'échantillon,
ii) le traitement du biomarqueur à base de glycane séparé avec de la N-glycosidase pour fournir le N-glycane, et
iii) la détermination du niveau de N-glycane du biomarqueur à base de glycane par spectrométrie de masse.

6. Procédé selon la revendication 3, dans lequel le niveau du au moins un biomarqueur à base de glycane dans ledit échantillon d'urine et/ou de salive et ledit niveau témoin sont déterminés à l'aide d'une ou plusieurs parmi la spectrométrie de masse, la liaison à une lectine, la liaison à un anticorps et la liaison à un aptamère.

7. Procédé selon la revendication 5, dans lequel l'échantillon est de l'urine, et la liaison à une lectine est déterminée à l'aide d'un réseau comprenant une ou plusieurs lectines choisies dans un groupe constitué de UDA, GRFT, CALSEPA, BANLEC, NPA (NPL, DL), HHA (HHL, AL), Con A, GAL1, BC2L-A, SAMB, ORYSATA, PALa, PTL-I (PTL, WBA-I), GS-I (GSL-I, BSL-I), PSA (PEA), LENTIL, LEA (LEL, TL), F17AG, LcH (LCA) et DISCOIDIN I, de préférence GAL1, ORYSATA, PALa, LENTIL, F17AG, LcH (LCA) et DISCOIDIN I.

8. Procédé selon la revendication 5, dans lequel l'échantillon est de la salive, et la liaison à une lectine est déterminée à l'aide d'un réseau comprenant une ou plusieurs lectines choisies dans un groupe constitué de PTL-1 (PTL, WBA-I), CNL, GAL7-S et CSA.

9. Procédé selon l'une quelconque des revendications 1 à 8, dans lequel le sujet est un enfant.

10. Procédé selon l'une quelconque des revendications 1 à 9, comprenant la réalisation d'au moins une procédure de neuroimagerie choisie dans un groupe constitué de radiographie, de tomodensitométrie et d'imagerie par résonance magnétique sur un sujet lorsque le niveau dudit au moins un biomarqueur à base de glycane dans un échantillon de fluide corporel dudit sujet est indicatif d'un TBI.

11. Utilisation d'au moins un biomarqueur à base de glycane comprenant un N-glycane choisi parmi H4N2F1, H3N2, H9N2, H4N3F1S1 et H4N3F351, dans laquelle
H4N2F1 est 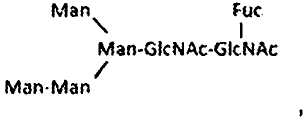
H3N2 est 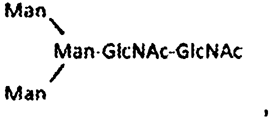
H9N2 est 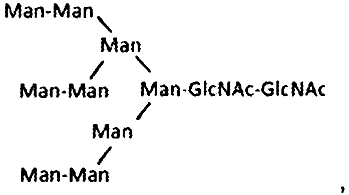
H4N3F1S1 est 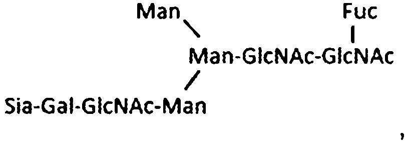 et
H4N3F3S1 est 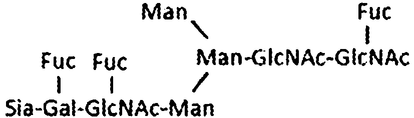
comme indicateur de traumatisme crânien dans l'urine.

12. Utilisation d'au moins un biomarqueur à base de glycane comprenant du N-glycane choisi dans un groupe constitué de H5N4F2P1, H5N4F3P1, H5N5F151 et H3N2F1, dans laquelle
H5N4F2P1 est choisi parmi 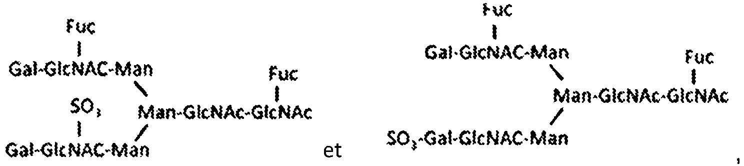
H5N4F3P1 est choisi parmi 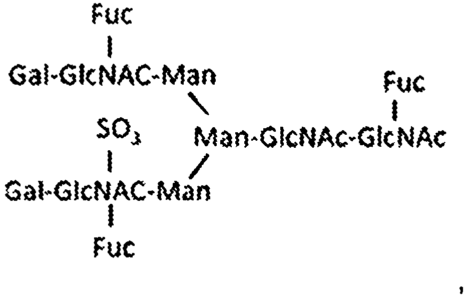 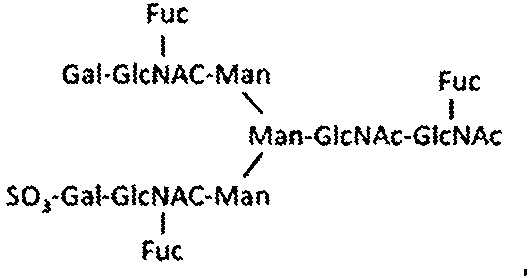 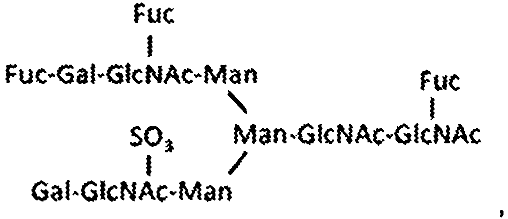 et 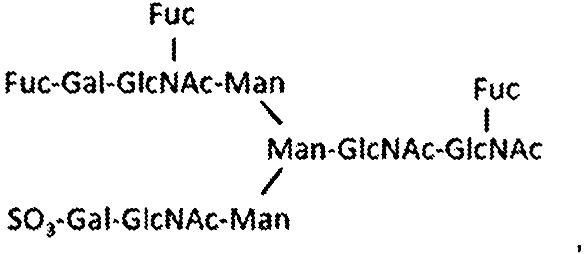
H5N5F1S1 est 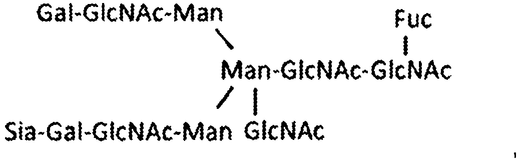 et
H3N2F1 est choisi dans le groupe constitué de
Manα1-3(Manα1-6)Manβ1-4GlcNAcβ1-4(Fucα1-6)GlcNAc,
Manα1-2Manα1-3Manβ1-4GlcNAcβ1-4(Fucα1-6)GlcNAc,
Manα1-3Manα1-6Manβ1-4GlcNAcβ1-4(Fucα1-6)GlcNAc, et
Manα1-6Manα1-6Manβ1-4GlcNAcβ1-4(Fucα1-6)GlcNAc, de préférence 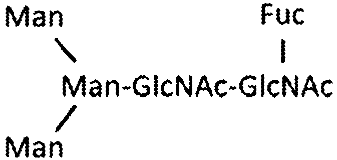
comme indicateur de traumatisme crânien dans la salive.
